(19) Europäisches Patentamt
European Patent Office
Office européen des brevets

(11) Veröffentlichungsnummer: **0 344 565**
**A1**

# (12) EUROPÄISCHE PATENTANMELDUNG

(21) Anmeldenummer: **89109180.3**

(22) Anmeldetag: **22.05.89**

(51) Int. Cl.[4]: **C07D 235/32 , C08G 63/76 , B27K 3/34 , B27K 3/50 , A01N 25/10 , A01N 47/18**

(30) Priorität: **30.05.88 AT 1398/88**

(43) Veröffentlichungstag der Anmeldung: **06.12.89 Patentblatt 89/49**

(84) Benannte Vertragsstaaten: **CH DE ES FR IT LI SE**

(71) Anmelder: **VIANOVA KUNSTHARZ AKTIENGESELLSCHAFT**
**Postfach 191 Leechgasse 21**
**A-8011 Graz(AT)**

(72) Erfinder: **Awad, Rami, Dipl.-Ing.**
**Papiermühlgasse 28**
**A-8020 Graz(AT)**
Erfinder: **Rauch-Puntigam, Harald, Dr.**
**Hochsteingasse 21**
**A-8010 Graz(AT)**
Erfinder: **Kriessmann, Ingo, Dr.**
**Hilmteichstrasse 75**
**A-8010 Graz(AT)**

## (54) Verfahren zur Herstellung von N-(2-Benzimidazolyl)-carbamidsäurealkylester-Präparationen für Holzschutzlacke.

(57) N-(2-Benzimidazolyl)-carbamidsäurealkylester-Präparationen für Holzschutzlacke mit verbesserter Lagerstabilität werden durch Umurethanisierung der Carbamidsäureester mit Verbindungen, die mindestens 2 Hydroxylgruppen tragen erhalten. Als Hydroxylverbindungen werden Diole, Polyole, Alkydharze oder partiell mit Diolen veresterte Adduktverbindungen eingesetzt. Die Fungizid-Polyolverbindungen können gegebenenfalls mit Fettsäuren, Alkydharzen oder Alkydharzrohstoffen verestert bzw. umgeestert werden. Die Reaktion erfolgt in Gegenwart von Organozinnverbindungen als Katalysator.

EP 0 344 565 A1

# Verfahren zur Herstellung von N-(2-Benzimidazolyl)-carbamidsäurealkylester-Präparationen für Holzschutzlacke

Alkylester der N-(2-Benzimidazolyl)-Carbamidsäure der Formel

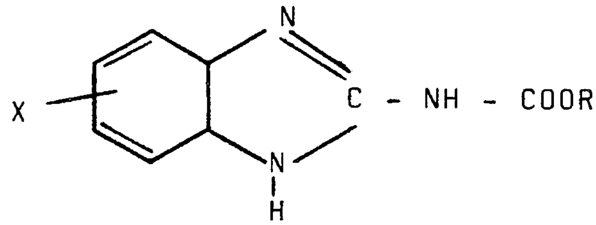

wobei R einen Alkylrest oder einen Alkoxyalkylrest und X ein Wasserstoff- oder ein Halogenatom oder eine Nitro-oder eine Alkylgruppe darstellt, sind aus der Literatur als Fungizide bzw. als Zwischenprodukte für die Herstellung von Fungiziden zur Bekämpfung von Schimmelpilzen, Bläuepilzen oder Moderfäule bekannt (DE-AS 20 40 069). Ihre Herstellung wird beispielsweise in der US-PS 3,637,733 beschrieben.

Ein wesentlicher Nachteil dieser Produkte ist bekannterweise ihre Schwerlöslichkeit in allen üblichen Lösemitteln, wie dies z. B. für das 2-(Methoxycarbonylamino)-benzimidazol (abgekürzt als MBC bezeichnet) in ULLMANN, Band 12. Seite 9/10 angegeben wird, wodurch ihre Verteilung im Anwendungsmedium sehr erschwert wird. Handelsprodukte dieses Fungizidtyps sind daher in der Regel Umsetzungsprodukte des MBC, wobei sich vor allem Reaktionsprodukte mit Mono- oder Diisocyanaten als besonders geeignet herausgestellt haben (siehe z. B. die US-PS 3,631, 176; die US-PS 3,970,668 oder die DE-OS 26 41 759). Wie in ULLMANN (s.o.) angegeben wird, zerfällt die Harnstoffverbindung - wahrscheinlich durch die Einwirkung von Feuchtigkeit - unter Rückbildung von MBC. Für spezielle Anwendungen, wie in Holzlasuren, Holzim prägnierungen u. ä., sind jedoch auch diese Anwendungsformen wenig geeignet, da die Verbindungen nur in speziellen Lösemitteln in ausreichendem Maße löslich und mit den eingesetzten Kunstharzen nicht verträglich sind.

In einer nicht zum Stand der Technik gehörenden Patentanmeldung (AT-A 41/87) wird ein Verfahren vorgeschlagen, wobei ein N-(2-Benzimidazolyl)-carbamidsäurealkylester über ein Diisocyanat an ein Polykondensations-oder ein Polyadditionsharz gebunden wird. Es hat sich jedoch gezeigt, daß zum Beispiel bei Verwendung von MBC bei längeren Lagerzeiten auch in diesem Fall die Harnstoffverbindung nicht stabil genug ist und die damit hergestellten Holzlasuren etc. zur Rückbildung des MBC und damit zum Trübwerden neigen.

Es wurde nun gefunden, daß es durch eine katalytisch unterstützte Umurethanisierung mit Polyhydroxylverbindungen möglich ist, lagerstabile Lösungen von N-(2-Benzimidazolyl)-carbamidsäurealkylester-Präparationen zu erhalten.

Die vorliegende Erfindung betrifft demgemäß ein Verfahren zur Herstellung von N-(2-Benzimidazolyl)-carbamidsäurealkylester-Präparationen, welches dadurch gekennzeichnet ist, daß man eine mindestens 2 Hydroxylgruppen tragende Verbindung partiell mit dem N-(2-Benzimidazolyl)-carbamidsäurealkylester, vorzugsweise dem N-(2-Benzimidazolyl)-carbamidsäuremethylester (MBC), bei 140 bis 200 °C in Gegenwart einer Organozinnverbindung, vorzugsweise Dibutylzinndilaurat, unter Abspaltung des Alkohols umsetzt und gegebenenfalls das Reaktionsprodukt einer weiteren (Poly)veresterung unterwirft.

Die Erfindung betrifft weiters die nach diesem Verfahren erhaltenen N-(2-Benzimidazolyl)-carbamidsäurealkylester-Präparationen, sowie deren Verwendung als Alleinbindemittel oder Fungizidzusatzmittel für Holzschutzlacke.

Die erfindungsgemäßen Präparationen zeichnen sich insbesonders dadurch aus, daß die Wirksamkeit des N-(2-Benzimidazolyl)-carbamidsäurealkylesters auch in der reagierten Form gegeben ist, da die fungizidwirksame Imidazolylgruppierung nicht blockiert wird.

Bei Verwendung von harzartigen Polyhydroxylverbindungen können die Präparationen auch unmittelbar als Bindemittel für die Holzschutzlacke eingesetzt werden.

Wie bereits erwähnt, werden als fungizid-wirksame N-(2-Benzimidazolyl)-carbamidsäurealkylester Verbindungen der allgemeinen Formel

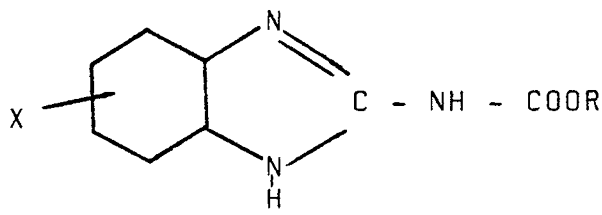

eingesetzt, wobei R einen Alkylrest oder einen Alkoxyalkylrest mit 1 bis 4 C-Atomen und X ein Wasserstoff- oder ein Halogenatom oder eine Nitro- oder eine Alkylgruppe darstellt. Für das erfindungsgemäße Verfahren wird vorzugsweise der N-(2-Benzimidazolyl)-carbamidsäuremethylester (2-(Methoxycarbonylamino)-benzimidazol, N-(2-Benzimidazolyl)-methylcarbamat) (abgekürzt als MBC bezeichnet) eingesetzt.

Als Hydroxylverbindung mit mindestens 2 Hydroxylgruppen für die Umsetzung mitdem N-(2-Benzimidazolyl)-carba midsäurealkylester können sowohl Diole oder Polyole als auch harzartige, Hydroxylgruppen aufweisende Polykondensationsprodukte oder mit Polyolen aufgeschlossene Maleinsäureanhydrid-Addukte verwendet werden.

Eine bevorzugte Gruppe von Polyolen sind die Di- und Trimethylolalkane, wie Neopentylglykol, Trimethylolethan oder -propan. Als harzartige Polykondensationsverbindungen können zur Umurethanisierung Hydroxylgruppen tragende Alkydharze herangezogen werden. Bevorzugt werden dabei die sogenannten langöligen Alkydharze (mit einem Fettsäureanteil (berechnet als Triglycerid) von mindestens 60 %) eingesetzt. Die Harze weisen eine Hydroxylzahl über 10 mg KOH/g auf. Als Polyadditionsverbindungen können vor allem die Maleinsäureanhydrid-Adduktverbindungen von längerkettigen, olefinisch ungesättigten Verbindungen, wie von trocknenden Ölen, von diese Öle aufbauenden ungesättigten Fettsäuren oder von niedermolekularen Dienpolymerisaten, wie handelsüblichen Polybutadienölen, herangezogen werden, wenn deren Anhydridgruppen zur Einführung von Hydroxylgruppen partiell mit Diolen aufgeschlossen werden. Vorteilhafterweise wird dabei zuerst ein Teil der Anhydridgruppen mit Wasser oder Monoalkoholen reagiert, um eine doppelseitige Reaktion der Diole möglichst hintanzuhalten.

Der Einbau der Fungizidkomponente in Harze kann auch durch Veresterung und/oder Umesterung der N-(2-Benzimidazolyl)-carbamidsäurealkylester-Polyolverbindung mit Alkydharzen oder Adduktverbindungen erfolgen. Durch Veresterung mit Fettsäuren kann die Harzverträglichkeit eines Additives auf Basis einer N-(2-Benzimidazolyl)-carbamidsäurealkylester-Polyolverbindung weiter verbessert werden. In allen Fällen kann der Anteil der Fungizidkomponente so gewählt werden, daß ein unmittelbar verwendbares Harz resultiert oder daß bei Vorliegen einer höheren Fungizidkonzentration eine Additivkomponente zur Abmischung mit anderen Bindemitteln vorliegt.

Die Umurethanisierung erfolgt bei 140 bis 200°C in Gegenwart einer Organozinnverbindung, vorzugsweise Dibutylzinndilaurat. Die maximale Menge an N-(2-Benzimidazolyl)-carbamidsäurealkylester hängt von der Anzahl der Hydroxylgruppen der Hydroxylverbindung ab. Die eingesetzten Mengen an N-(2-Benzimidazolyl)-carbamidsäurealkylester liegen zwischen 0,1 und 20 Gew.-%, bei Verwendung von Alkydharzen als Polyhydroxylkomponente vorzugsweise bei 0,1 bis 5 Gew.-%.

Die eingesetzten Harze können entweder in organischen Lösemitteln löslich sein oder bei entsprechendem Aufbau nach Neutralisation ihrer ionenbildenden Gruppen wasserlöslich sein. Für den Einsatz als Holzschutzanstriche werden in vielen Fällen wasserverdünnbare Produkte bevorzugt.

Die Formulierung, Herstellung und Anwendung der Holzschutzanstriche, wie Lasuren oder Imprägnierungen sind dem Fachmann bekannt bzw. der Fachliteratur zu entnehmen.

Die folgenden Beispiele erläutern die Erfindung, ohne sie in ihrem Umfang zu beschränken. Alle Angeben in Teilen oder Prozenten beziehen sich, soferne nichts anderes angegeben ist, auf Gewichtseinheiten.

Beispiel 1:Umurethanisierung von MBC mit einem Alkydharz

In 1000 g eines handelsüblichen Alkydharzes (ca. 78 % Öllänge, Basis Tallölfettsäuren, Säurezahl ca. 6 mg KOH/g, Hydroxylzahl 25 mg KOH/g) werden 12 g MBC (1,2 Gew.-% entsprechend 63 Millimol) und 0.5 g Dibutylzinndilaurat bei 80°C unter Schutzgas eingerührt. Anschließend wird die Temperatur auf 180°C gesteigert, bis die theoretische Methanolmenge abdestilliert ist und aus der ursprünglichen Suspension eine klare Lösung entstanden ist. Das Produkt wird mit Lackbenzin auf einen Festkörpergehalt von 80 % verdünnt. Weder die Harzlö sung noch eine in üblicher Weise hergestellte Holzlasur zeigen nach sechsmonatiger Lagerung eine Trübung.

Beispiel 2: Umurethanisierung von MBC mit einem Polyol

230 g Trimethylolpropan, 50 g MBC und 0,2 g Dibutylzinndilaurat werden bei 70 bis 80 °C vorsichtig aufgeschmolzen und anschließend langsam auf 160 °C und weiter innerhalb von 60 Minuten auf 180 °C erhitzt. Diese Temperatur wird gehalten, bis die theoretische Methanolmenge abgeschieden ist.

Beispiel 3:Veresterung der MBC-Polyolverbindung mit einer Fettsäure

Die gemäß Beispiel 2 hergestellte MBC-Polyolverbindung wird mit 1000 g Tallölfettsäure bei 200 °C in Gegenwart von Xylol als Azeotroplösungsmittel verestert, bis eine Säurezahl von unter 5 mg KOH/g erreicht ist. Das Lösungsmittel wird unter Vakuum abdestilliert. Das Produkt weist einen Fungizidgehalt von 4 % auf und wird als Additiv für Alkydharze, sowie gegebenenfalls wäß rige Alkydharzemulsionen eingesetzt. Die mit dem Konzentrat hergestellten Holzschutzlacke zeigen nach sechsmonatiger Lagerung keine Trübung.

Beispiel 4:MBC-Präparation auf Adduktbasis für wasserlösliche Systeme

Aus 100 g Trimethylolpropan und 50 g MBC wird in Gegenwart von 0,2 g Dibutylzinndilaurat wie in Beispiel 2 eine MBC-Polyolverbindung hergestellt, welche mit einem Maleinatöl bei 60 bis 70 °C bis zu einer Säurezahl von 40 mg KOH/g verestert wird. Das Addukt wurde durch vollständige Bindung von 8 Gew.-% Maleinsäureanhydrid an ein 1 : 1-Gemisch aus Leinöl und dehydratisiertem Rizinusöl bei 180 bis 200 °C hergestellt. Es weist eine Säurezahl von 91 mg KOH/g auf.

Die Fungizidpräparation ist nach Neutralisation von ca. 80 % der freien Carboxylgruppen mit Triethylamin oder Ammoniak mit Wasser unbegrenzt verdünnbar. Eine 25%ige wäßrige Lösung ist opak und zeigt auch nach 6 Monaten keine Ausfällungen. Der MBC-Gehalt beträgt 4 %. Die Lösungen können beispielsweise als Einlaßgrund Verwendung finden.

## Ansprüche

1. Verfahren zur Herstellung von N-(2-Benzimidazolyl)-carbamidsäurealkylester-Präparationen, dadurch gekennzeichnet, daß man eine mindestens 2 Hydroxylgruppen tragende Verbindung partielle mit dem N-(2-Benzimidazolyl)-carbamidsäurealkylester, vorzugsweise dem N-(2-Benzimidazolyl)-carbamidsäuremethylester (MBC), bei 140 bis 200 °C in Gegenwart einer Organozinnverbindung, vorzugsweise Dibutylzinndilaurat, unter Abspaltung des Alkohols umsetzt und gegebenenfalls das Reaktionsprodukt einer weiteren (Poly)-veresterung unterwirft.

2. Verfahren nach Anspruch 1, dadurch gekennzeichnet, daß man als Hydroxylgruppen tragende Verbindung ein Diol oder ein Polyol, vorzugsweise ein Di- oder Trimethylolalkan, einsetzt.

3. Verfahren nach Anspruch 1, dadurch gekennzeichnet, daß man als Hydroxylgruppen tragende Verbindung ein Alkydharz mit einer Hydroxylzahl von mehr als 10 mg KOH/g einsetzt.

4. Verfahren nach Anspruch 3, dadurch gekennzeichnet, daß man als Hydroxylgruppen tragende Verbindung ein Alkydharz einsetzt, welches einen Fettsäureanteil (berechnet als Triglycerid) von mehr als 60 % aufweist.

5. Verfahren nach Anspruch 1, dadurch gekennzeichnet, daß man als Hydroxylgruppen tragende Verbindung eine partiell mit Diolen, sowie Wasser und/oder Monoalkoholen halbveresterte Additionsverbindung aus Maleinsäureanhydrid und längerkettigen olefinisch ungesät tigten Verbindungen, vorzugsweise trocknenden Ölen, einsetzt.

6. Verfahren nach den Ansprüchen dadurch gekennzeichnet, daß man das N-(2-Benzimidazolyl)-carbamidsäurealkylester/Polyol-Reaktionsprodukt mit Fettsäuren oder den üblichen Alkydharzrohstoffen oder einer Additionsverbindung aus Maleinsäureanhydrid und längerkettigen olefinisch ungesättigten Verbindungen, vorzugsweise trocknenden Ölen (poly)verestert.

7. Verfahren nach den Ansprüchen 1 bis 5, dadurch gekennzeichnet, daß man das Umsetzungsprodukt aus N-(2-Benzimidazolyl)-carbamidsäurealkylester und der mindestens 2 Hydroxylgruppen tragenden Verbindung durch Umesterung in ein Alkydharz einbaut.

8. N-(2-Benzimidazolyl) -carbamidsäurealkylester-Präparationen, hergestellt nach dem Verfahren gemäß den Ansprüchen 1 bis 7.

9. Verwendung der nach den Ansprüchen 1 bis 7 hergestellten N-(2-Benzimidazolyl)-carbamidsäurealkylester-Präparationen als Alleinbindemittel oder Fungizidzusatzmittel für Holzschutzlacke.

Europäisches Patentamt

# EUROPÄISCHER RECHERCHENBERICHT

Nummer der Anmeldung

EP 89 10 9180

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | CHEMICAL ABSTRACTS Band 84, Nr. 23, 7. Juni 1976, Seite 469, Spalte 2, Zusammenfassung Nr. 164780y, Columbus, Ohio, USA; & JP - A - 75 142565 (YOSHITOMI PHARMACEUTICAL INDUSTRIES LTD.) 17.11.1975 in Zusammenhang mit CHEMICAL SUBSTANCE INDEX, Band 84, C-H, Januar-Juni 1976, Seite 1245CS, Spalte 2, Zeilen 54,69 --- | 8 | C 07 D 235/32<br>C 08 G 63/76<br>B 27 K 3/34<br>B 27 K 3/50<br>A 01 N 25/10<br>A 01 N 47/18 |
| A | EP-A-0 048 368 (BAYER AG) Anspruch 1; Seite 8, Zeile 20 - Seite 9, Zeile 29 * --- | 1 | |
| A | EP-A-0 077 497 (DR. WOLMAN GMBH) * Ansprüche * --- | 9 | |
| A | EP-A-0 014 443 (RIEDEL-DE HAEN AG) * insgesamt * --- | 1,9 | |
| A | US-A-4 267 281 (C. L. MC CORMICK) * Spalte 4, Zeile 56 - Spalte 5, Zeile 14 * --- | 1 | **RECHERCHIERTE SACHGEBIETE (Int. Cl.4)** |
| A | US-A-4 496 724 (C. L. MC CORMICK) * Spalte 4, Zeilen 1-24 * --- | 1 | C 07 D 235/00<br>C 08 G 63/00<br>B 27 K 3/00<br>A 01 N 25/00<br>A 01 N 47/00 |
| A | US-A-4 565 856 (S. I. TROTZ et al.) ----- | | |

Der vorliegende Recherchenbericht wurde für alle Patentansprüche erstellt

| Recherchenort | Abschlußdatum der Recherche | Prüfer |
|---|---|---|
| BERLIN | 02-08-1989 | VAN AMSTERDAM L.J.P. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsatze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)